# EUROPEAN PATENT SPECIFICATION

(11) **EP 4 048 073 B1**
(45) Date of publication and mention of the grant of the patent: **23.04.2025**
(21) Application number: 20793388.8
(22) Date of filing: 23.10.2020
(51) Int. Cl.: A01P 1/00, A01N 63/50, A01N 43/72

(54) **FIBRINOGEN AS ADJUVANT FOR ANTIMICROBIAL AGENTS AND THERAPY**
FIBRINOGEN ALS ADJUVANS FÜR ANTIMIKROBIELLES MITTEL UND THERAPIE
FIBRINOGÈNE EN TANT QU'ADJUVANT POUR AGENT ANTIMICROBIEN ET THÉRAPIE

(30) Priority: 23.10.2019 EP 19204958
(43) Date of publication of application: 31.08.2022
(73) Proprietor: Fibriant B.V., 2333 CG Leiden (NL)
(72) Inventor: KOOPMAN, Jacob, 2333 CG Leiden (NL); WEGGEMAN, Miranda, 2333 CG Leiden (NL); GRIMBERGEN, Joseph, 2333 CG Leiden (NL)
(74) Representative: Morabito, Sara
(86) International application number: PCT/EP2020/079970
(87) International publication number: WO 2021/078986

(56) References cited:
- WO-A1-2019/207016
- WO-A1-96/17633
- WO-A2-2011/093932
- WO-A2-2014/135689
- FR-A1- 2 938 544
- US-A1- 2015 166 735
- NIS JØRGENSEN ET AL: "Streptokinase Treatment Reverses Biofilm-Associated Antibiotic Resistance in Staphylococcus aureus", MICROORGANISMS, vol. 4, no. 3, 20 September 2016 (2016-09-20), pages 36, XP055758700, ISSN: 2076-2607, DOI: 10.3390/microorganisms4030036
- TELMA BLANCA LOMBARDO BEDRAN ET AL: "Fibrinogen-Induced Streptococcus mutans Biofilm Formation and Adherence to Endothelial Cells", BIOMED RESEARCH INTERNATIONAL, vol. 2013, 1 January 2013 (2013-01-01), pages 1 - 8, XP055758682, ISSN: 2314-6133, DOI: 10.1155/2013/431465

## Description

### Technical field

The present invention relates to a method for increasing the sensitivity of microorganisms for antimicrobial agents and therapies, in particular it may be used to reduce the resistance of biofilms against antimicrobials

### Background of the Invention

Antimicrobial resistance of pathogens is a cause for growing concern around the world. Frequently, pathogens have managed to escape from the action of once powerful antimicrobial compounds such as penicillins. Different strategies to overcome the problem of antimicrobial resistance are used : 1) new antimicrobials are being developed , 2) combination therapy, using two or more existing antimicrobials, is being used and 3) therapeutic strategies and products that serve as antimicrobial adjuvants are used in conjunction with existing antimicrobial drugs to reduce the resistance of the pathogen to this antimicrobial drug. Antimicrobial adjuvant products and therapeutic strategies include anti resistance drugs (e.g. beta-lactamase inhibitors) , anti-virulence drugs ( e.g. bacterial toxin inhibitors) and host-directed therapies (e.g. innate immune system agonists).

One of the mechanisms that microorganisms employ to increase the resistance against antimicrobial agents and therapies is to form aggregates, microcolonies or biofilms. Pathogens in biofilms or aggregates are known to be 10 to 1000 fold more resistant to antimicrobial agents than free-floating pathogens in planktonic form. This is a serious problem in the field of medical devices, when biofilms form on indwelling medical devices, such as catheters, stents, heart valves, pacemakers and joint replacements and cause 65% of all infections. Biofilm formation may lead to failure of the device and to spread of infections associated with the device. Removal of the device followed by or in combination with antibiotic treatment and replacement of the device after eradication of the infection is now the recommended approach. Alternatives for removal and replacement have been suggested. Kretlow et al. (2014) Plast Reconstr Surg. 2014, 133(1):28e-38e describe in situ treatment of left ventricular assist devices with antibiotic beads.

A further complication is the existence of polymicrobial biofilms that contain certain fungi and viruses or fungi and bacteria resulting in increased resistance to anti-viral, anti-bacterial and anti-fungal agents.

Since adhesion of pathogens is required for colonization and for subsequent development of disease, several have focused on preventing adhesion of the pathogen to the surface to prevent the formation of a biofilm. Fibrinogen, being one of the most abundant host plasma proteins, is considered to be an important host proteins to promote the formation of biofilms. Flores-Mireles et al. J Urol 2016: 416 describes fibrinogen depositions as accumulation surface for uropathogens. Kwiecinski et al. (J. Infect. Dis. 2016:213) describes how fibrinolytic enzymes prevent formation of biofilms.

Fibrinogen is also considered to increase biofilm resistance to antibiotics. Bedran et al. (Biomed Research International vol. 2013, article ID 431465) reports *in vitro* results of fibrinogen induced biofilm formation by *S. mutans* with increased resistance to penicillin. Jorgensen et al. (Microorganisms 2016: 4) describes how fibrinolytic enzymes which specifically degrade fibrinogen or fibrin significantly increase the susceptibility to antibiotics of bacterial biofilms *in vitro.* These publications show that *in vitro* the presence of fibrinogen stimulates biofilm formation and that fibrinolysis, which lead to depletion of fibrinogen, improves the efficacy of antibiotics in the treatment of biofilm infections.

### Brief description of the drawings

**Figure 1** Adhesion of *S. aureus* to different immobilized fibrinogen species.
**Figure 2** Viable *S. aureus* bacteria associated with biofilms formed *in vitro* on surfaces pre-coated with different fibrinogen species.
**Figure 3** Viable *S. aureus* bacteria detected in biofilms formed *in vitro* on non-pre-coated surfaces with plasma Fib or rhFib Gamma prime in the bacterial culture medium during biofilm formation.
**Figure 4** Viable *S. aureus* bacteria detected in biofilms formed *in vivo* on PTFE cages pre-coated with plasma Fib or rhFib Gamma prime, implanted under the skin of mice and infected with *S. aureus* bacteria.
**Figure 5** Viable *S. aureus* bacteria detected in biofilms formed *in vitro* on surfaces pre-coated with different fibrinogen species and subsequently exposed to different dosages of vancomycin or ampicillin.
**Figure 6** Viable *S. aureus* bacteria detected in biofilms formed *in vitro* on non-pre-coated surfaces in the presence of plasma Fib (**6A** and **6C**) or rhFib Gamma prime(**6B** and **6D**) in the bacterial culture medium; **6E)** 50/50 mixture of plasma Fib and rhFib Gamma prime subsequently exposed to vancomycin.
**Figure 7** Viable *S. aureus* bacteria detected in biofilms formed *in vivo* on PTFE cages pre-coated with plasma Fib, rhFib WT or rhFib Gamma prime, implanted under the skin of mice, after therapeutic (**7A**) and prophylactical (**7B**) daptomycin treatment.

### Detailed Description of the Invention.

The present invention relates to the use of a composition comprising fibrinogen for treating or preventing antimicrobial resistance in an individual.

The use according to the invention alleviates the problem of resistance of microorganisms to antimicrobial agents. In particular, it increases the susceptibility of microorganisms for antimicrobial therapy and is particularly advantageous to combat microorganism which are typically minimally susceptible to antimicrobial compounds, such as microorganisms in biofilms, in aggregates or in microcolonies. Biofilms, which are a community of microorganisms, are notoriously resistant to antimicrobial agents, i.e. these biofilms escape from the action of once powerful antimicrobial compounds. The composition for use according to the invention may serve as antimicrobial adjuvant in conjunction with existing antimicrobial drugs to reduce the resistance of microbial biofilms or aggregates to these antimicrobial drugs.

In one embodiment, a composition for use according to the invention increases the sensitivity of bacteria for anti-bacterial compounds. Anti-bacterial compounds are compounds which stop the growth of bacteria (bacteriostatic) or kill bacteria (bactericidal) and include antibiotics, bacteriophages and their endolysines, antibacterial or antimicrobial peptides and antibacterial antibodies. The skilled person will understand that a bactericidal compound for some bacteria may be bacteriostatic to other bacteria, and the other way around. Preferably, the anti-bacterial compound is a natural, synthetic or semi-synthetic antibiotic. Suitable natural, synthetic or semi-synthetic antibiotics include aminoglycosides, antibacterial imidazoles, beta lactams, macrolides, peptide antibiotics, polyclonal or monoclonal antibodies, quinolones, sulphonamides and tetracyclines, and derivatives thereof.

Examples of aminoglycosides are gentamycin and tobramycin. Examples of antibacterial imidazoles are miconazole and metronidazole; Examples of beta-lactams are penicillins, including natural penicillins, aminopenicillins, beta-lactamase-resistant penicillins, carboxypenicillins, such as amoxicillin, ampicillin and penicillin; cephalosporins, such as cephalexim; and carbapenems, such as meropenem and doripenem. Examples of macrolides are ketolide and macrolide antibiotics, such as clarithromycin, erythromycins and telithromycin. Examples of peptide antibiotics are glycopeptide antibiotics, such as vancomycin, and lipopeptide antibiotics, such as daptomycin. Examples of antibodies are antibodies directed against *S. aureus* clumping factor A, alpha toxin, lipoteichoic acid and teichoic acid. Examples of quinolones are fluoroquinolones, such as ciprofloxacin and levofloxacin. Examples of sulphonamides are sulfacetamide and sulfamethoxazole. Examples of tetracyclines are tetracycline and doxycycline. In one embodiment, the anti-bacterial compound is a penicillin or a peptide antibiotic. In another embodiment, the anti-bacterial compound is a carboxypenicillin, a glycopeptide antibiotic or a lipopeptide antibiotic. In yet another embodiment, the anti-bacterial compound is selected from the group consisting of ampicillin, vancomycin, daptomycin and tobramycin.

The skilled person will be familiar with the fact that some antimicrobial agents are used against several groups of microorganisms. For example, some antimicrobial peptides may be used both against bacterial and viral or fungal infections.

The composition may also be used to treat infections comprising several types of microorganisms. The composition may be used to increase antimicrobial susceptibility of one type of microorganism or several types of microorganisms. For example, viruses or yeasts in a bacterial biofilm may be rendered more susceptible to antiviral or anti-yeast agents.

Infectious microorganisms in biofilms or aggregates may also be referred to as pathogens. A notorious pathogen in biofilm-associated infections which may be treated using the adjuvant method of the invention is the bacterium *Staphylococcus aureus* in particular, methicillin-resistant *S. aureus* (MRSA).

Compositions for use according to the invention increase the antimicrobial susceptibility of microorganisms. Microorganisms which are treated according to the invention are preferably in cluster form, such as in aggregates or clumped together, or they may be adhered to a surface, for example in a biofilm. Microorganisms in cluster form or in biofilm tend to be less susceptible to antimicrobial agents than microorganisms in free floating form. In a biofilm, microorganisms are typically surrounded by an extracellular matrix from polymeric substances (EPSs), such as glycoproteins, secreted by the microorganisms themselves or recruited form the host. Biofilm is also referred to in the art as the glycocalyx.

Surfaces of attachment include living surfaces, such as tissues, cell surfaces and wound beds, and inert surfaces, such as, medical devices, particularly indwelling medical devices or implants, including catheters, cardiac implantable electronic devices (CIED), heart valves, immobilizers, joint replacements, stents, tracheostomies and wound drains. Examples of catheters are peripherally inserted central catheters (PICC lines), implantable ports and urinary catheters. Examples of CIED are pace makers, left ventricular assist devices (LVADs) and implantable defibrillators. Examples of immobilizers are pins, plates, rods, screws, and wires. Examples of joint replacements are hip, knee and shoulder prostheses. The devices may comprise or consist of metal, ceramics or non-biodegradable synthetic polymers, like plastics or polytetrafluorethylene (PTFE, Teflon (trademark)), or biodegradable polymers, like collagen, hyaluronic acid, polylactic acid or poly urethane. The medical devices are preferably indwelling devices or implants, which are meant to stay in the body for several days, weeks, months or years.

The composition for use according to the invention comprises fibrinogen. The fibrinogen in the composition may be plasma fibrinogen (plasma Fib) which is a mixture of all the different fibrinogen variants that occur in the blood of healthy individuals; or it may be a preparation from one or more of these specific fibrinogen variants either isolated from plasma (or plasma fibrinogen) or produced by recombinant protein production technology. The fibrinogen in the composition preferably comprises human plasma fibrinogen, human WT fibrinogen, a human fibrinogen gamma prime, a human fibrinogen alpha extended variant or a human fibrinogen alpha truncated variant. In one embodiment, the fibrinogen in the composition consists of human plasma fibrinogen, human WT fibrinogen, a human fibrinogen gamma prime variant, a human fibrinogen alpha extended variant or a human fibrinogen alpha truncated variant. Human fibrinogen refers to fibrinogen having the amino acid sequence as found in the majority of human beings.

Plasma fibrinogen refers to fibrinogen isolated from plasma, typically comprising a mixture of naturally occurring fibrinogen variants that are the result of alternative splicing, proteolytic degradation or post-translational modifications. Plasma fibrinogen may have been derived from one individual or may be pooled from more individuals. Preferably, the plasma fibrinogen is of high purity, i.e., comprised in a composition which comprises at least 95% w/w, at least 97% w/w or at least 98% w/w plasma fibrinogen. Purity may be determined by clottability.

WT fibrinogen refers to the mature, dominant form of human fibrinogen representing 60-80% of the total plasma fibrinogen in circulation, having Aα chains of 610 amino acids, Bβ chains of 461 amino acids and γ chains of 411 amino acids. In the literature this form of fibrinogen is also referred to as High Molecular Weight (HMW) fibrinogen or intact fibrinogen.

Fibrinogen gamma prime variants have two alpha polypeptide chains, two beta polypeptide chains and two gamma polypeptide chains, wherein at least one of the gamma polypeptide chains is a gamma prime polypeptide chain. In humans, a gamma prime polypeptide chain has 427 amino acids. If a fibrinogen molecule comprises one WT gamma polypeptide chain (γ411) and one gamma prime polypeptide chain (γ427), this variant is referred to here as fibrinogen gamma prime heterodimer or Fib γ427/411. If both gamma polypeptide chains are of the gamma prime type (γ427), this variant is referred to as fibrinogen gamma prime homodimer or Fib γ427/427. The compositions for use according to the invention may comprise or consist of mixtures of gamma prime variants, such as a mixture of fibrinogen gamma prime heterodimer and homodimer, or it may comprise or consist of one type of gamma prime variant.

Fibrinogen alpha extended variants have two alpha polypeptide chains, wherein at least one of the alpha polypeptide chains is an alpha extended polypeptide chain, two beta polypeptide chains and two gamma polypeptide chains, wherein at least one of the gamma polypeptide chains is a gamma prime polypeptide chain. In humans, an alpha extended polypeptide chain has 847 amino acids. If a fibrinogen molecule comprises one WT alpha polypeptide chain (Aα 610) and one alpha extended polypeptide chain (Aα 847), this variant is referred to here as fibrinogen alpha extended heterodimer or rhFib Aα 847/Aα 610. If both alpha polypeptide chains are of the alpha extended type (Aα 847), this variant is referred to as fibrinogen alpha extended homodimer or Fib Aα 847/847. The compositions for use according to the invention may comprise or consist of mixtures of alpha extended variants, such as a mixture of fibrinogen alpha extended heterodimer and homodimer, or it may comprise or consist of one type of alpha extended variant.

Fibrinogen alpha truncated variants have two alpha polypeptide chains wherein at least one of the alpha polypeptide chains is truncated between the amino acid at position 251 and the carboxyl terminal amino acid at position 610, two beta polypeptide chains and two gamma polypeptide chains. If a fibrinogen molecule comprises one WT alpha chain (Aα 610) and one truncated alpha polypeptide chain (Aα <610 and ≥ 251), this variant is referred to here as fibrinogen alpha truncated heterodimer or Fib Aα610/truncated ≥ 251 (also referred to in the literature as LMW fibrinogen when isolated from plasma). If both alpha polypeptide chains are of the alpha truncated type (Aα <610 and ≥ 251), this variant is referred to as fibrinogen alpha truncated homodimer or Fib Aα truncated (also known in the literature as LMW' fibrinogen when isolated from plasma). Amino acid sequences for fibrinogen have been published, see for example WO2010/004004 for sequences of the alpha, beta and gamma chain and variants thereof.

The compositions for use according to the invention may comprise or consist of mixtures of alpha truncated variants, such as a mixture of fibrinogen alpha truncated heterodimer and homodimer, or it may comprise or consist of one type of alpha truncated variant.

Fibrinogens combining alpha chain variants (extended or truncated) with gamma prime variants including fibrinogen that are heterodimer for both variant polypeptide chains, hetero dimer for alpha or gamma variants and that are homo dimer for both variant polypeptide chains may be used in compositions for use according to the invention.

The fibrinogen in the composition may be obtained by methods known in the art. For example, plasma fibrinogen may be obtained by isolation from plasma or it may be obtained commercially, for example from Enzyme Research Labs, Swansea, United Kingdom as FIB 3 or Fib 1. Preferably, purified plasma fibrinogen is used, for example at least 95% clottable plasminogen depleted fibrinogen. Specific fibrinogen variants may be obtained by purification from plasma or from commercially available plasma fibrinogen. Alternatively, specific fibrinogen variants may be obtained by recombinant production, for example by cloning or chemical synthesis of the encoding genomic or cDNA followed by transfection using a host cell or cell culture system, such as a mammalian or human cell culture system. Recombinant production of proteins has many advantages over the use of plasma derived materials. These include its preferred safety profile, the possibility to make variants in a pure way and the fact that there is an unlimited supply.

In order to produce it in an economically feasible way, high expression levels of intact, functional fibrinogen or its variants are required. In addition, for specific applications, proper post-translational modifications (e.g. glycosylation) are required. Therefore, in one embodiment of the invention, for pharmaceutical standards, the fibrinogen is preferably produced in a mammalian cell culture system, such as in baby hamster kidney (BHK) cells, NSO cells, Sp2/0 cells, PER.C6 cells, HEK293 cells, insect cell, Chinese Hamster Ovary (CHO) cells or African Green Monkey derived COS cells. In a preferred embodiment, the mammalian fibrinogen in the composition for use according to the invention is produced in CHO cells.

In a preferred embodiment, the fibrinogen in the composition for use according to the invention consists of human fibrinogen gamma prime, preferably in its homodimer form and preferably recombinantly produced.

In another preferred embodiment, the fibrinogen in the composition for use according to the invention consists of recombinant human fibrinogen alpha extended preferably in its homodimer form.

In another preferred embodiment, the fibrinogen in the composition for use according to the invention consists of recombinant human fibrinogen combining a gamma prime polypeptide chain with an alpha extended polypeptide chain, preferably in its homodimer form for both the gamma and alpha variant polypeptide chains.

The compositions may be used to pre-coat a medical device for implantation before implantation, or coat a wound surface before dressing or closing. (Pre)coating with the composition increases the sensitivity of the bacteria for antibiotics *in vivo,* in comparison with non-coated surfaces. The coating may be thin, such as a film, or thick, comprising several layers. The composition may be formulated as an aerosol, drops, dry powder, emulsion, foam, gel, ointment, paste, salve, semi-gel, spray or suspension. Preferably, the composition is formulated as a spray, paste or gel.

The coating may be continuous or discontinuous. Pre-coating of medical devices may be performed by incubation for several hours at 37° C with a fibrinogen solution containing 10 - 100 microgram/ml fibrinogen in PBS or by spraying a solution with a concentration of 100-1000 microgram/ml at room temperature, optionally in combination with a thrombin solution. Coating of a wound or tissue surface in situ may be performed by spraying a fibrinogen solution containing 1-50 mg/ml, optionally in combination with a thrombin solution. Typically 0.1-1 ml of coating solution per cm² of coating surface will suffice. To ensure a homogeneous efficacy of the final coating across the whole surface of the medical device or cell tissue, the composition is preferably distributed evenly over a given width of the medical device or tissue, so that the fibrinogen concentration per area unit of the surface to be coated varies at most 50%, such as at most 25%, such as at most 10%. In one embodiment, a coating solution comprises at least 1 microgram/ml, at least 5 microgram/ml, at least 10 microgram/ml, at least 25 microgram/ml or at least 50 microgram/ml fibrinogen.

The composition may be applied to the medical device or wound tissue using an applicator, a dressing, wound packing, bandage or swab.

The sensitivity or susceptibility of microorganisms for antimicrobial compounds is enhanced by the method according to the invention. Enhanced sensitivity may be reflected in several ways, such as by slower growth or by cell death, which may be apparent, for example, from lower dry weight, a decrease in colony forming units (CFU), a decrease in metabolic activity, compared to a control situation in which the composition is not used. In one embodiment, the decrease is between 1 log and 5 log reduction in CFUs. In another embodiment, in mice experiments, increased antibiotic sensitivity is clear from a reduction of 6 log or more to 0 CFU when the composition is used, to only a reduction of 3 log- if the composition is not used. Cure rate may be increased by at least 25%, compared to a control without fibrinogen. In another embodiment, cure rate was increased by 5% to 100% or 5% to 60% or 60% to 100%.

Bacteria which may be rendered more susceptible to antibiotics in this way are Gram positive or Gram negative, anaerobic, facultative anaerobic or aerobic. Suitable examples of bacteria include bacteria belonging to the genera *Acinetobacter, Bacillus, Bacteroides, Burkholderia, Campylobacter, Clostridium, Enterococcus, Escherichia, Klebsiella, Listeria, Mycobacterium, Neisseria, Pseudomonas, Salmonella, Yersinia, Staphylococcus* and *Streptococcus.* In particular, species which are infamously antibiotic resistant and have low antibiotic susceptibility, such as *Acinetobacter baumannii, Bacillus, Bacteroides, Burkholderia cepacia, Campylobacter, Clostridium difficile, Enterococcus faecalis, Escherichia coli, Klebsiella pneumoniae, Listeria, Mycobacterium tuberculosis, Neisseria gonorrhoeae, Pseudomonas aeruginosa, Salmonella, Yersinia, Staphylococcus aureus, Staphylococcus epidermidis, Streptococcus mutans* and *Streptococcus pyogenes.*

Preferably, the bacteria belong to the genera *Pseudomonas, Staphylococcus* or *Streptococcus.* More preferably, the bacteria are strains of *Staphylococcus aureus,* including ampicillin-resistant *S. aureus,* vancomycin resistant *S. aureus,* methicillin-resistant *S. aureus,* such as *S. aureus* 300 WT or *S. aureus* 43300, oxacillin-resistant *S. aureus* and multi-resistant *S. aureus.*

The composition for use according to the invention may advantageously be used for treatment or prevention of any infection, referring to an invasion or multiplication of microorganisms which are not normally present or which are present in abnormal amounts within an individual's body. The infection may be subclinical, without symptoms, or it may be clinically, with apparent symptoms. Infections which may be treated or prevented include bacteremia, bone infection, chest infections, endocarditis, epidural abscesses, genital infections, meningitis, osteoarticular infections, peritonitis, respiratory tract infections, such as pneumonia; prosthetic joint infections, skin infections, such as acne and rosacea; septicaemia, septic thrombophlebitis, toxic shock syndrome, urinary tract infections and wound infection.

The fibrinogen composition according to the invention may include a therapeutically effective amount or a prophylactically effective amount of fibrinogen. A therapeutically effective amount refers to an amount effective, at dosages and for periods of time necessary, to achieve the desired therapeutic result. A therapeutically effective amount of a variant fibrinogen or compositions thereof may be determined by a person skilled in the art and may vary according to factors such as the disease state, age, sex, and weight of the individual, and the ability of the mammalian fibrinogen or composition to elicit a desired response in the individual. A therapeutically effective amount is also one in which any toxic or detrimental effects of variant fibrinogens or compositions of the invention are outweighed by the therapeutically beneficial effects. A prophylactically effective amount refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The fibrinogen content of the composition for pre-coating of devices is preferably from 0.0001% w/w to 0.1 w/w, such as from 0.001% w/w to 0.05% w/w, from 0.001% w/w to 0.1% w/w or from 0.005% w/w to 0.1% w/w, by weight of the composition. The fibrinogen content of the composition for coating of wound or tissue surfaces is preferably from 0.01% w/w to 10% w/w, such as from 0.1% w/w to 5% w/w, by weight of the composition.

In another embodiment of the invention, the fibrinogen composition according to the invention is a pharmaceutical composition or preparation, preferably sterile. Therefore, the composition for use according to the invention may further comprise a pharmaceutically-acceptable excipient, such as an anti-adherent, antioxidant, binder, bulking agent, carrier, colourant, disintegrant, diluent, filler, flavour, lubricant, preserving agent, solvent, surfactant, sweetener, vehicle or wetting agent. The excipient should not adversely affect the stability of the composition in the formulation. The term "pharmaceutically acceptable" refers to suitable for preparing a pharmaceutical composition, such as generally considered safe and non-toxic.

Some examples of materials which can serve as pharmaceutically acceptable excipients include buffering agents such as, but not limited to, magnesium hydroxide and aluminium hydroxide; alginic acid; pyrogen-free water; isotonic saline; Ringer's solution; ethyl alcohol, and phosphate buffer solutions, as well as other nontoxic excipients, such as sodium lauryl sulphate and magnesium stearate, as well as preservatives and antioxidants, which may also be present in the composition. Techniques and formulations may generally be found in "Remington's Pharmaceutical Sciences," (Meade Publishing Co., Easton, Pa.). Preferably the carrier, vehicle or diluent includes sterile water, sterile isotonic saline, sterile Ringer's solution or sterile lactate solution, the selection of which would be well known to those skilled in the art. Suitable solvents include water, isotonic saline, ethyl oleate, glycerine, hydroxylated castor oils, alcohols such as ethanol, and phosphate buffer solutions. The amount of solvent(s) in a systemic composition may range from 80% to about 99.95% w/w or w/v.

The composition for use according to the invention may further comprise an activator, such as thrombin or a thrombin-like enzyme, such as reptilase or batroxobin, or a non-enzymatic activator such as a peptide that binds fibrinogen. The thrombin and thrombin-like enzymes convert fibrinogen monomers in to fibrin polymers by cleaving the fibrinopeptides A or B from the alpha chain and beta chain respectively. The cleaved fibrin(ogen) monomers polymerize spontaneously into a double stranded protofibril that can grow latterly and form fibrin polymers that make up the 3-dimensional fibrin network. Preferably, the activator is human thrombin, most preferably in purified form, isolated from plasma or recombinantly produced.

The non-enzymatic activator is a substance which comprises at least 2 fibrinogen binding sites and by binding to 2 different fibrinogen molecules (which has 2 activator binding sites because fibrinogen is a symmetrical dimer) introduces a polymer form of fibrinogen that has properties similar to thrombin-induced fibrin.

The composition for use according to the invention may serve as antimicrobial adjuvants and may be used in conjunction with an antimicrobial to reduce the resistance of a pathogen to this antimicrobial. The antimicrobial may be applied separately from or together with the composition comprising fibrinogen. If applied separately, antimicrobial and fibrinogen may be applied at the same time, or before or after each other. If the antimicrobial and fibrinogen are applied together, they may be present in separate compositions or combined in one composition. Therefore, compositions for use according to the invention may also further comprise one or more antimicrobials, including antibiotic agents, anti-fungal agents, anti-yeast agents and antiviral agents. In one embodiment, the composition for use according to the method of the invention is supplied together with an antibiotic, separately or in the same composition.

The composition may be used for increasing antimicrobial susceptibility in any individual in need thereof, such as humans and mammalian animals, including alpaca, bison, buffalos, cats, cows, deer, dogs, donkeys, horses, mice, pigs, rabbits and sheep. For treatment of animals, typically the animal equivalent of human fibrinogen is preferably used.

In another aspect, the present invention relates to a medical device coated with fibrinogen, in particular indwelling devices or implants, which are meant to stay in the body for several days, weeks, months or years. Examples of medical devices are mentioned above and include catheters, endotracheal tubes, enteral feeding tubes, heart valves, joint replacements, stents, tracheostomies and wound drains. The device may be supplied or commercially obtained pre-coated, or may be pre-coated just before use, such as a few hours, minutes or seconds before use.

In yet another aspect, the present invention relates to a method for increasing antimicrobial susceptibility of microorganisms. In one embodiment, the method comprises providing a composition comprising fibrinogen to a human or animal individual to prevent or treat an infection by a microorganism in the individual, optionally in conjunction with an antimicrobial to reduce the resistance of the microorganism to the antimicrobial. In another embodiment, the method comprises applying a fibrinogen composition to a medical device or to a wound bed. In one embodiment, the fibrinogen composition is applied *ex vivo* to a medical device, before implantation or introduction of the medical device in the body of a human or animal individual. The composition comprising fibrinogen may be any of the compositions which have been described above for use according to the invention.

The skilled person will understand that embodiments and preferred embodiments mentioned for use of the composition may also be applied to the medical device and to the method for increasing antimicrobial susceptibility of microorganisms, and vice versa.

### EXAMPLES

### Materials & Methods

### Bacteria

Methicillin Resistant *Staphylococcus aureus* (MRSA) strains USA 300 and USA 43300 were obtained from ATTCC and routinely grown in Tryptic Soy Broth (TSB) or on Tryptic Soy Agar (TSA) plates medium at 37°C.

### Fibrinogen

Purified plasma fibrinogen (plasma Fib) was obtained from Enzyme Research Labs, Swansea, United Kingdom. The rhFib WT and rhFib Gamma prime and rhFib Alpha extended and rhFib Gamma prime/Alpha extended were made by standard recombinant DNA protein production technology. cDNA sequences encoding the fibrinogen Aα610 (WT) chain, Aα 847 (Alpha extended) chain, Bβ chain, y411 (Gamma) chain and y427 (Gamma prime) chain were cloned in the pcDNA 3.1 plasmid (Invitrogen, Carlsbad, CA, USA) to construct expression vectors for the different fibrinogen chains. Combinations of expression plasmids containing cDNA 's encoding Aα, Bβ and γ chains were used to produce the different fully assembled recombinant human species by transient expression using HEK 293 cells (Life technologies EXPI293 system) according to the manufacturer's instructions or by stable expression in CHO cells. CHO cells were grown in ProCHO5 cell culture medium (Westburg, Leusden, The Netherlands) and were transfected using electroporation with an Amaxa device (Westburg, Leusden, The Netherlands) according to the manufacturer's instructions. Using blasticidin and zeocin as selection markers, clones producing fibrinogen were selected using an ELISA method described earlier (Hoegeede Nobel E. et al. Thrombosis Haemostasis 1988 Dec22; 60(3): 415-8). Selected CHO clones were grown in CD FortiCHO cell culture media (Life Technologies Europe, Bleiswijk, The Netherlands) for 7 to 10 days at 37°C and under 5% CO2. Cell supernatant was collected by centrifugation or filtration and recombinant fibrinogen variants were purified using affinity purification on a GPRP column essentially as described in Kuyas C et al. (Thrombosis Haemostasis 1990 Jun 28; 63 (3): 439-444) and formulated in phosphate buffered saline (PBS) pH 7.4 at concentrations between 2 -40 mg/ml.

### Resazurin method

The Resazurin method, for measuring cell metabolic activity, was performed as described by Lee A. and Jain E. Tip Biosystems Application note AN016 Rev. 1.0 Mar 2017.

In brief, the Resazurin based Biofilm susceptibility assay was performed as follows. A working stock of USA 300 *S. aureus* containing 1×10⁸ CFU/mL (OD₆₀₀ approx. 0.1 AU) was prepared by growing the bacteria in TSB. Fibrinogen dilutions were prepared in PBS pH 7.4. 90 µL of TSB and 90 µL of fibrinogen dilution were added to 96-well tissue culture microtiter plates. Subsequently 20µL of *S. aureus* working stock was added to each well and incubated at 37° for 24h to establish a biofilm. Plates were washed three times with 0.9% sterile saline and 100µL of Cation-adjusted Mueller Hinton Broth (CAMHB) with varying concentrations of antibiotic (0-100 µg/mL) were added to the 96-well plates and incubated at 37° for 24h. 10µL (10% of sample volume) of alamarBlue (resazurin) solution was added to each well, gently shaken and incubated for 1- 4h at 37° C. The absorbance at 570 and 600nm was measured to determine cell viability.

### Example 1: In vitro adhesion of S. aureus to different fibrinogen species

Binding experiments are performed essentially as described earlier (Flick M. et al Blood 2013 Mar 7; 121(10): 1783-94). In brief, the binding assays are performed as follows: 96 well microtiter plates were coated with fibrinogen solutions in phosphate buffered saline (PBS) (0-25 µg/ml) by overnight incubation at 37°C. After washing and blocking, wells are incubated for 2 hours at 37°C with a *S. aureus* USA 300 suspension in PBS (OD 600 nm approx. 0.4). After washing, the plates are incubated with 0.1% crystal violet solution for 30 min and after washing, the bound crystal violet is solubilized in 10% acetic acid and the absorbance at 570 nm is measured to quantify the bacterial adhesion.

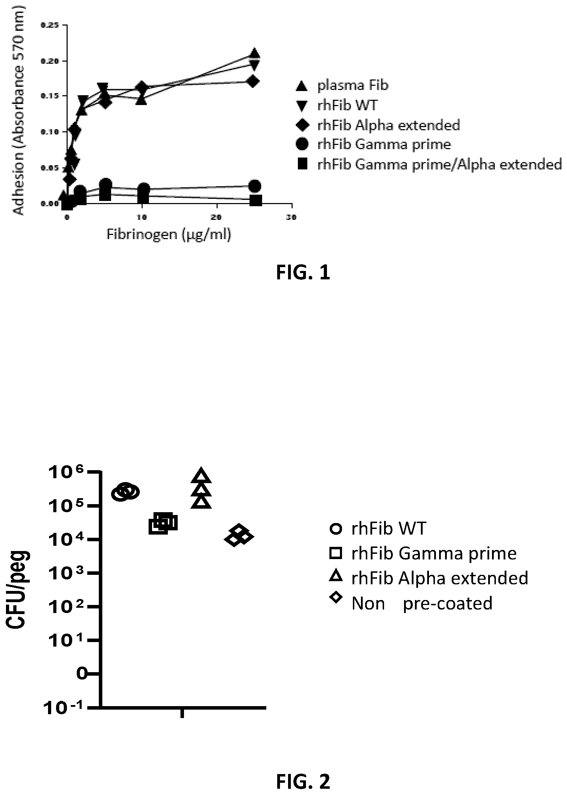

Fig 1 shows that *S. aureus* bacteria bind to purified plasma Fib, rhFib WT and rhFib Alpha extended but not to rhFib Gamma prime or rhFib Alpha extended/Gamma prime. This indicates that adhesion of *S. aureus* to fibrinogen is dependent on the presence of the carboxyl -terminus of the fibrinogen gamma chain (y411) that contains the AGDV motif. The different length of the alpha chain (Aα610 *vs* Aα847) has no influence on the *S. aureus* binding to fibrinogen. These results are in line with literature data.

### Example 2 In vitro biofilm formation on surfaces pre-coated with different fibrinogen species.

The *in vitro* biofilm formation on pre-coated polystyrene surfaces was described earlier (Harrison JJ. et al. 2010 Nature Protocols 5, 1236-1254) and performed using the Innovotech (Edmonton, Alberta, Canada) MBEC assay according to the manufacturers protocol.

In brief, the assay was performed as follows. Polystyrene lids of 96 well microtiter plates with 96 pegs were pre-coated by submerging the pegs in 0.25 ml of solution containing different fibrinogen species dissolved in PBS buffer at a concentration of 10 or 100 microgram/ml for 24 hours at 37° C. After washing the pre-coated pegs (and non-pre-coated controls) were incubated for 24 hours at 35°C with a bacterial suspension of ^{~}10E6 CFU/ml in TSB + 1% glucose to allow biofilm formation. After washing the pegs to remove planktonic bacteria, the pegs with the biofilm were incubated with CAMHB for 24 hours at 37°C. After washing, the viable bacteria were detached from the surface by the mild sonification and the number of viable bacteria was determined by plating out on TSA plates. Colonies were counted to determine the CFU/peg surface. Results are shown in Fig 2.

A small reduction in viable bacteria of approximately 1 log is observed on non-pre-coated controls and on rhFib Gamma prime pre-coated pegs as compared to pegs coated with rhFib WT or rhFib Alpha extended.

These results are surprising in view of the very significant differences in binding observed in Example 1 (and literature) that demonstrate a strong dependence of binding on the availability of the carboxyterminus of fibrinogen containing the AGDV binding motif. The results suggest that the number of viable bacteria incorporated into biofilms is only slightly dependent on the interaction with the pre-coated fibrinogen.

In conclusion, *in vitro* pre-coating of surfaces with fibrinogen does not significantly increase or reduce the number of viable bacteria in the biofilms formed on such surfaces.

### Example 3: In vitro biofilm formation on non-pre-coated surfaces in the presence of different fibrinogen species in the bacterial culture media.

The effect of different fibrinogen species in solution present during the biofilm formation on surfaces (in contrast to pre-coating surfaces before biofilm formation) was determined using the Resazurin assay described above.

Purified plasma Fib or rhFib Gamma prime at concentrations between 0-250 µg/ml were added to TSB followed by inoculation of this mixture with bacteria and subsequent incubation for 24 hours at 37°C to allow coating of fibrinogen and biofilm formation to take place simultaneously on polystyrene surfaces. The amount of viable bacteria in the biofilms formed under the different conditions was compared by measuring the OD 570 nm after addition of resazurin.

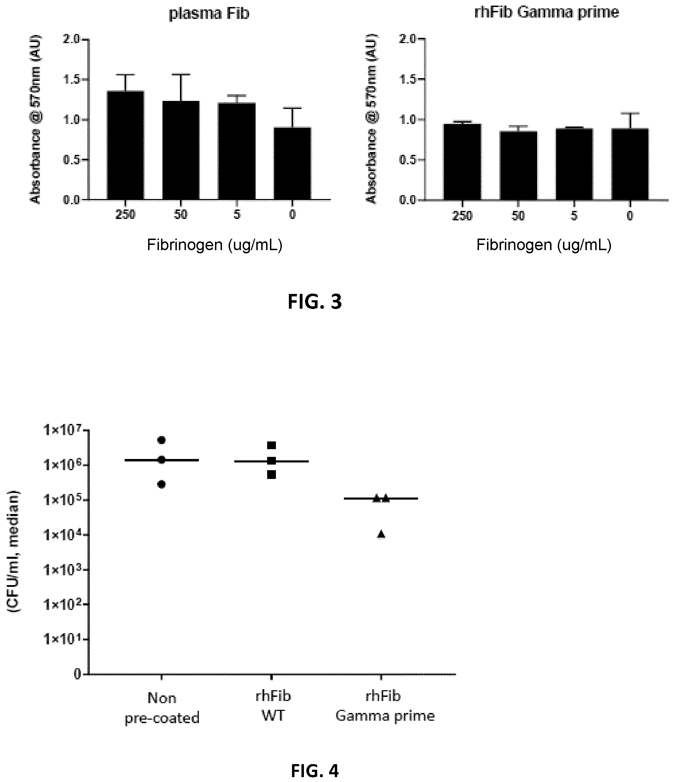

The results are shown in Fig 3 and show that the amount of viable bacteria in the biofilms was not significantly different under all conditions. However, a small increase in viable bacteria was observed in the biofilms formed in the presence of plasma Fib as compared to no fibrinogen. These results are very similar to the results obtained in Example 2, using surfaces pre-coated with fibrinogen and confirm that the amount of viable bacteria associated with biofilms is largely independent of fibrinogen.

### Example 4: In vivo biofilm formation on pre-coated surfaces

The *in vivo* effect of pre-coating with different fibrinogen species on biofilm formation by *S. aureus* USA 43300 was performed essentially as described earlier (Nowakowska J et al. Antibiotics 2014, 3, 378-397) In brief the study was performed as follows.

Polytetrafluorethylene cages, pre-coated by incubation for 24 hours at 37 degrees C with a fibrinogen solution in PBS containing 10 or 100 microgram/ml rhFib WT or rhFib Gamma prime and non-pre-coated controls, were implanted under the skin of C57BI mice. The cages were infected with approx. 400 CFU *S. aureus* /cage. Two days after implantation the cages were removed and adherent viable bacteria were measured using mild sonification to detach the bacteria from the surface followed by plating out on TSA plates and colonies were counted to determine the number of CFU per cage.

The results in Fig 4 show that there was no difference in the amount of CFU between non-pre-coated cages and cages pre-coated with rhFib WT. A small, clinically non-significant decrease (approx. 1 log) in the amount of CFU in cages coated with rhFib Gamma prime as compared to non-pre-coated and rhFib WT pre-coated cages was observed.

These results show that *in vivo* the number of adherent bacteria in the biofilms formed on non-pre-coated cages and on cages pre-coated with rhFib WT were virtually identical, whereas the cages pre-coated with rhFib Gamma prime contained a slightly lower number (approx.1 log) of adherent bacteria.

These *in vivo* results are in line with the *in vitro* results in Examples 2 and 3 and support the finding that the number of viable adherent bacteria in biofilms is to a large extent independent of pre-coating with fibrinogen.

### Example 5: In vitro analysis of antibiotic sensitivity of biofilms formed on pre-coated surfaces.

To determine the effect of pre-coating surfaces with different fibrinogen species on the antibiotic sensitivity of the bacteria in the biofilm, *in vitro* experiments were performed as described in Example 2. During the incubation step with CAMBH different concentrations (0 to 100 µg/ml) of vancomycin or ampicillin were present. After antibiotic incubation, the surfaces were treated with mild sonification to release bacteria from the surfaces and CFU associated with the surfaces were determined.

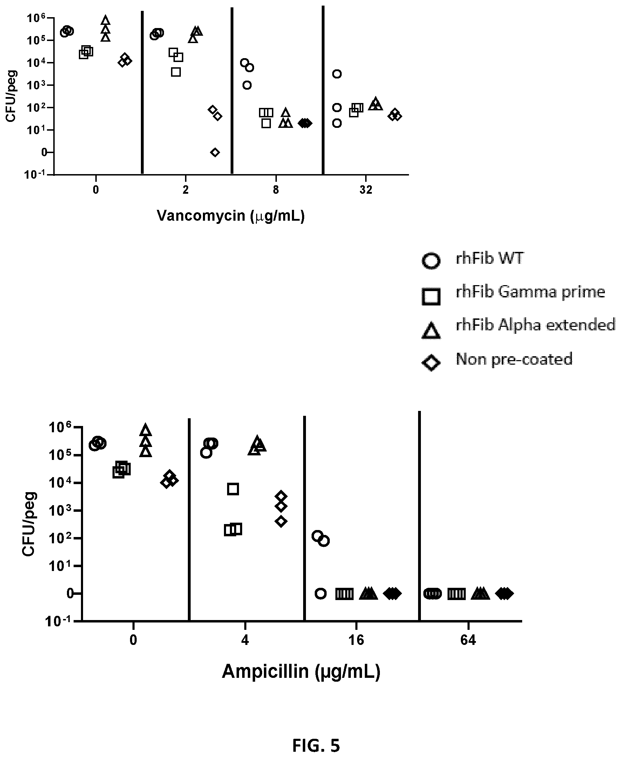

Results are shown in Fig 5 and show that *S. aureus* USA 300 biofilms formed on non-pre-coated pegs or pegs pre-coated with rhFib Gamma prime or rhFib Alpha extended are more sensitive to vancomycin and ampicillin exposure as compared to biofilms formed on pegs pre-coated with rhFib WT.

These results indicate that biofilms formed on non-pre-coated pegs or on pegs pre-coated with rhFib Gamma prime or rhFib Alpha extended are more susceptible to antibiotics than biofilms formed on rhFib WT pre-coated pegs. In other words, *in vitro,* antibiotic sensitivity of biofilms is increased by using rhFib Gamma prime and rhFib Alpha extended.

### Example 6: In vitro analysis of antibiotic sensitivity of biofilms formed on non-pre-coated surfaces

To determine the effect of the presence in solution during biofilm formation of different fibrinogen species and mixtures thereof on the antibiotic sensitivity of the bacteria in the biofilm, *in vitro* experiments were performed as described in example 3 using the Resazurin assay as described above. The percentage of viable cells as compared to the control, which control comprised 0 µg/ml antibiotic, was calculated from the OD measurements at 570 and 600 nm.

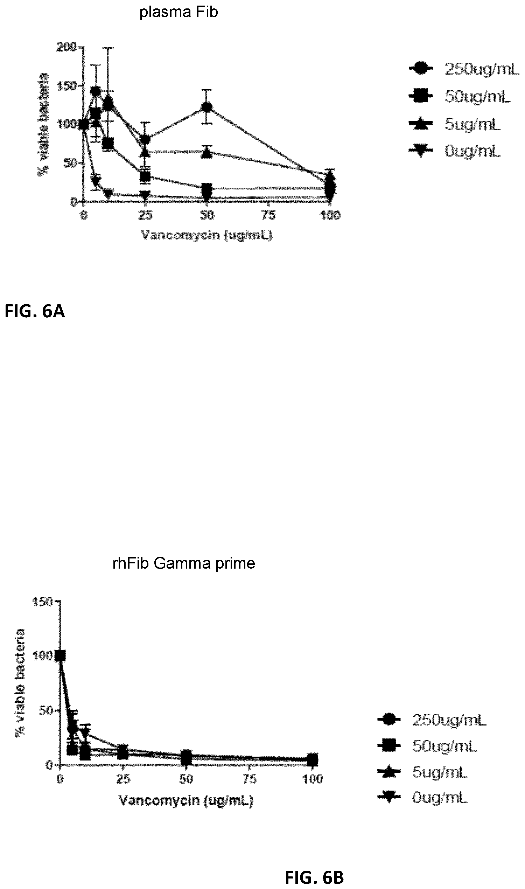

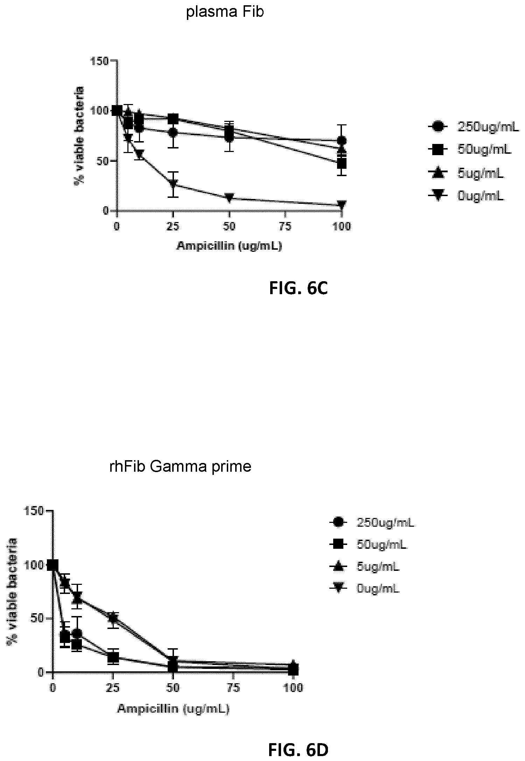

For purified plasma Fib, the vancomycin results in Fig. 6A show more viable bacteria in comparison to the control. This indicates that resistance in the biofilm for vancomycin is increased in comparison to the control, if plasma Fib is present. Similar results were observed for ampicillin (Fig 6C).

For rhFib Gamma prime, the vancomycin results in Fig. 6B show similar amounts off viable bacteria in comparison to the control. This indicates that resistance in the biofilm for vancomycin is not changed in comparison to the control if rhFib Gamma prime is present. With ampicillin (Fig 6D), higher rhFib Gamma prime concentration result in less viable bacteria as compared to control. This shows that, *in vitro,* susceptibility for ampicillin of an ampicillin-resistant strain may be increased by using rhFib Gamma prime.

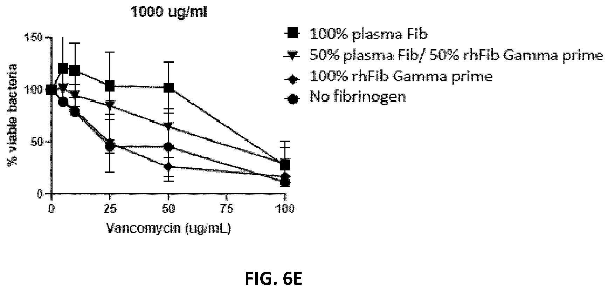

The results in Fig 6E show that biofilms formed *in vitro* on non-pre-coated surfaces with a 50/50 mixture of plasma Fib/rhFib Gamma prime at 1 mg/ml in the bacterial culture medium and treated with vancomycin contain significantly less viable *S. aureus* bacteria as compared to surfaces pre-coated with plasma Fib only. This result indicates that rhFib Gamma prime is able to increase antibiotic susceptibility, also in the presence of WT fibrinogen, which renders it clinically relevant.

### Example 7: In vivo analysis of antibiotic sensitivity of biofilm on pre-coated surfaces

To determine the *in vivo* effect of pre-coating surfaces with different fibrinogen species on the antibiotic sensitivity of the bacteria in the biofilm a study in mice was performed as described in example 4 with the addition of prophylactic and therapeutic daptomycin treatment. Polytetrafluorethylene cages, non-pre-coated or pre-coated with 10 or 100 µg/ml purified plasma Fib , rhFib WT or rhFib Gamma prime were implanted under the skin of C57BI mice.

Mice in the prophylactic group were treated with daptomycin (50 mg/kg ) 30 minutes before implantation and infection. Mice in the therapeutic were treated with daptomycin (50 mg/kg) 24 hours after implantation and infection. Mice were infected with a dose of 400-600 CFU/cage) of *S .aureus* 43300. At 48 hours after infection, the cages were explanted, washed and treated with ultrasound to release bacteria from the cage surface. CFU associated with the cages were determined.

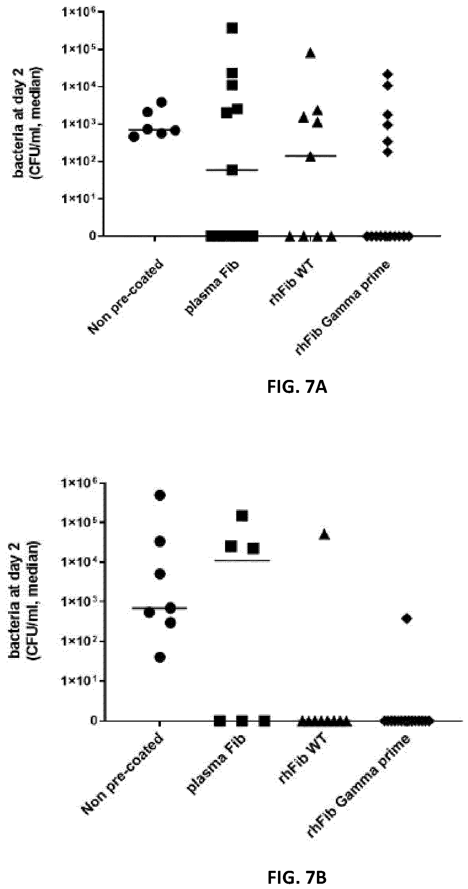

The results of therapeutic treatment (Fig. 7A) and prophylactic treatment (Fig. 7B) clearly show that all (100%) of the non-pre-coated cages (control) contained significant amounts of viable planktonic and adherent *S. aureus* bacteria despite the prophylactic or therapeutic treatment with daptomycin. This indicates that a biofilm formed *in vivo* on a non-pre-coated surface is highly resistant to antibiotic treatment. This is in contrast to the *in vitro* results of example 5 that biofilms formed *in vitro* on non-pre-coated surfaces were sensitive to antibiotic treatment. Clearly, *in vitro* results, do not have predicative value for *in vivo* results.

Without wishing to be bound by theory, an explanation for this discrepancy could be that in the *in vivo* experiment host extracellular proteins (e.g. fibronectin) adhere to the non-pre-coated cages that are implanted and that this result in a biofilm which renders the bacteria resistant to antibiotic treatment.

Only 50-60% of the cages pre-coated with plasma Fib (10 and 100 µg/ml coatings) contained viable adherent *S. aureus* bacteria after therapeutic (Fig 7A) or prophylactic (Fig 7B) treatment with daptomycin. This indicates that the biofilm formed *in vivo* on surfaces pre-coated with plasma Fib only provides partial resistance to antibiotic treatment. A similar result, 56-67 % cages containing viable bacteria, is observed with cages pre-coated with rhFib WT (10 and 100 µg/ml) and treated therapeutically (Fig 7A) with daptomycin. These results seem to be in contrast to the *in vitro* finding in example 5, which could be due to synergy with host defense mechanisms *in vivo.*

The cages pre-coated with rhFib Gamma prime (10 and 100 µg/ml) showed the strongest reduction in viable bacteria after prophylactic (Fig 7B) or therapeutic (Fig 7A) treatment with daptomycin. Fig 7B shows that only 7% of the cages pre-coated with rhFib Gamma prime and treated prophylactically with daptomycin contained viable bacteria (non-pre-coated cages 100%). When therapeutic daptomycin treatment was used (Fig 7A) 37% of the cages pre-coated with rhFib Gamma prime contained viable bacteria (non-pre-coated 100% and plasma Fib/ rhFib WT coated 55-67%). This surprisingly strong *in vivo* effect ( 5-6 log reduction) of rhFib Gamma prime pre-coating on the sensitivity of the bacteria for antibiotic treatment in a biofilm seems in line with the *in vitro* results (example 5 ) that show an increased sensitivity to antibiotics for bacteria in biofilms formed *in vitro* on rhFib Gamma prime pre-coated surfaces. However, the effect observed *in vivo* is much more pronounced as compared to the *in vitro* effect.

The data in Fig 7A and B suggest that pre-coating of medical devices or coating tissue surfaces with fibrinogen increases the sensitivity of biofilm associated bacteria for bactericidal treatments, such as antibiotic treatment. The results with rhFib Gamma prime are more pronounced.

### Example 8: In vivo analysis of antibiotic sensitivity of biofilms of different Staphylococci.

To determine the *in vivo* effect of pre-coated surfaces with different fibrinogen species on the antibiotic sensitivity of biofilms formed by several *Staphylococci,* a study in mice was performed as described in example 7 with prophylactic addition of daptomycin. One of the species included was *S. epidermidis,* which binds fibrinogen through its beta chain. The beta chain in rh Fib WT and in rhFib gamma prime is the same.

Polytetrafluorethylene cages, pre-coated with 10 or 100 µg/ml purified plasma Fib , rhFib WT or rhFib Gamma prime were implanted under the skin of C57BI mice. Non-coated cages were taken as a control. Mice were treated with daptomycin (50 mg/kg) 30 minutes before implantation and infection. The bacterial dose was 400-600 CFU/cage. At 48 hours after infection, the cages were explanted, washed and treated with ultrasound to release bacteria from the cage surface. CFU associated with the cages were determined. The results are presented in Table 1 and show that pre-coating with any type of fibrinogen works better than no pre-coat.

**Table 1**

| **% of mice free from viable adherent bacteria** | | | |
|---|---|---|---|
| | ***S. aureus* ATCC 43300** | ***S. aureus* ATCC 1556** | ***S. epidermidis* ATCC 35984** |
| daptomycin (50 mg/kg ) | | | |
| **pre-coating** | | | |
| none | 0 | 0 | 0 |
| plasma fib | 50 | 71 | n.a. |
| rh Fib WT | 89 | n.a. | 40 |
| rhFib gamma prime | 93 | 100 | 75 |

The results show that bacteria in biofilms show an increased sensitivity, and are thus less resistant, to antibiotics when the biofilm surface is pre-coated with fibrinogen, compared to no coating. Best results are obtained when the surface is pre-coated with rhFib Gamma prime, also for *S. epidermidis* biofilms.

### Example 9 In vivo analysis of antibiotic sensitivity of Pseudomonas biofilms

Example 8 was repeated with *Pseudomonas* aeruginosa strain PA01 with prophylactic addition of tobramycin, to determine the *in vivo* effect of pre-coating surfaces with fibrinogen on the antibiotic sensitivity of biofilms of Gram negative bacteria. *Pseudomonas aeruginosa* is a Gram negative bacterium with no known binding site for fibrinogen. Tobramycin (50 mg/kg) instead of daptomycin was used, since tobramycin is typically used as an antibiotic against Gram negative bacteria. The results are presented in Table 2 and show that pre-coating with fibrinogen works better than no pre-coating. The Gram negative bacteria in the biofilm show an increased sensitivity to antibiotics when the biofilm surface is pre-coated with fibrinogen, compared to none coating.

**Table 2**

| | **% of mice free from viable adherent *P.aeruginosa*** |
|---|---|
| | tobramycin (50 mg/kg ) |
| **pre-coating** | |
| none | 50 |
| rhFib gamma prime | 100 |

## Claims

1. A composition comprising fibrinogen for use in a method for treating or preventing antimicrobial resistance of microorganisms in a human or animal individual.

2. The composition for use according to claim 1, wherein the antimicrobial is an antibacterial agent, preferably an antibiotic.

3. The composition for use according to any preceding claim, wherein the microorganisms are in a biofilm or in an aggregated form and the composition is for increasing antimicrobial susceptibility of the biofilm or aggregated form.

4. The composition for use according to any preceding claim, wherein the biofilm is on a medical device or wound tissue.

5. The composition for use according to any preceding claim, wherein the composition comprises or consists of plasma fibrinogen, wild type fibrinogen, fibrinogen gamma prime, fibrinogen alpha extended variant or fibrinogen alpha truncated variant.

6. The composition for use according to any preceding claim, wherein the fibrinogen is used to coat a medical device or implant before implantation, or a wound before dressing or closing.

7. The composition for use according to any preceding claim, the composition further comprising thrombin, batroxobin or reptilase.

8. The composition for use according to any preceding claim, wherein the method further comprises supplying an antimicrobial agent.

9. The composition for use according to claim 8, wherein the antimicrobial is included in the composition.

10. The composition for use according to any preceding claim, wherein the fibrinogen in the composition is produced recombinantly.

11. The composition for use according to any preceding claim, wherein the microorganisms are Gram positive bacteria or Gram negative bacteria.

12. The composition for use according to claim 11, wherein the bacteria are *Pseudomonas* species or *Staphylococci, preferably Staphylococcus aureus* species, more preferably ampicillin-resistant *S. aureus,* vancomycin resistant *S. aureus,* methicillin-resistant *S. aureus* or multi-resistant *S. aureus* strains.

13. The composition for use according to any preceding claim, wherein the composition is a spray, paste or gel.

14. The composition for use according to any of claims 4-13, wherein the medical device is a cardiac implantable electronic device (CIED), catheter, endotracheal tube, enteral feeding tube, heart valve, immobilizer, joint replacement, stent, tracheostomy, vascular access device or wound drain.

15. The composition for use according to any of claims 4-14, wherein the medical device comprises or consists of a biodegradable polymer, such as collagen, hyaluronic acid, polylactic acid or poly urethane.

## Patentansprüche

1. Fibrinogen enthaltende Zusammensetzung zur Verwendung in einem Verfahren zur Behandlung oder Vorbeugung einer antimikrobiellen Resistenz von Mikroorganismen in einem menschlichen oder tierischen Individuum.

2. Zusammensetzung zur Verwendung nach Anspruch 1, wobei das Antimikrobiotikum ein antibakterielles Mittel, vorzugsweise ein Antibiotikum, ist.

3. Zusammensetzung zur Verwendung nach einem der vorhergehenden Ansprüche, wobei die Mikroorganismen in einem Biofilm oder in einer aggregierten Form sind und die Zusammensetzung zur Erhöhung antimikrobieller Empfindlichkeit des Biofilms oder der aggregierten Form dient.

4. Zusammensetzung zur Verwendung nach einem der vorhergehenden Ansprüche, wobei sich der Biofilm auf einer medizinischen Vorrichtung oder Wundgewebe befindet.

5. Zusammensetzung zur Verwendung nach einem der vorhergehenden Ansprüche, wobei die Zusammensetzung Plasmafibrinogen, Wildtyp-Fibrinogen, Fibrinogen-Gamma-Prime, Fibrinogen-Alpha-erweiterte Variante oder Fibrinogen-Alpha-verkürzte Variante enthält oder daraus besteht.

6. Zusammensetzung zur Verwendung nach einem der vorhergehenden Ansprüche, wobei das Fibrinogen verwendet wird, eine medizinische Vorrichtung oder ein Implantat vor Implantation oder eine Wunde vor Verbinden oder Schließen zu beschichten.

7. Zusammensetzung zur Verwendung nach einem der vorhergehenden Ansprüche, wobei die Zusammensetzung ferner Thrombin, Batroxobin oder Reptilase enthält.

8. Zusammensetzung zur Verwendung nach einem der vorhergehenden Ansprüche, wobei das Verfahren ferner Bereitstellung eines antimikrobiellen Mittels enthält.

9. Zusammensetzung zur Verwendung nach Anspruch 8, wobei das antimikrobielle Mittel in der Zusammensetzung enthalten ist.

10. Zusammensetzung zur Verwendung nach einem der vorhergehenden Ansprüche, wobei das Fibrinogen in der Zusammensetzung rekombinant hergestellt ist.

11. Zusammensetzung zur Verwendung nach einem der vorhergehenden Ansprüche, wobei die Mikroorganismen grampositive Bakterien oder gramnegative Bakterien sind.

12. Zusammensetzung zur Verwendung nach Anspruch 11, wobei die Bakterien Pseudomonas-Arten oder Staphylokokken sind, vorzugsweise Streptococcus-aureus-Arten, insbesondere ampicillinresistente S. aureus-, vancomycinresistente S. aureus-, methicillinresistente S. aureus- oder mehrfachresistente S. aureus-Stämme.

13. Zusammensetzung zur Verwendung nach einem der vorhergehenden Ansprüche, wobei die Zusammensetzung ein Spray, eine Paste oder ein Gel ist.

14. Zusammensetzung zur Verwendung nach einem der Ansprüche 4 bis 13, wobei die medizinische Vorrichtung eine kardiale implantierbare elektronische Vorrichtung (CIED), ein Katheter, ein Endotrachealtubus, eine enterale Ernährungssonde, eine Herzklappe, ein Immobilisator, ein Gelenkersatz, ein Stent, eine Tracheotomie, eine Gefäßzugangsvorrichtung oder ein Wunddrain ist.

15. Zusammensetzung zur Verwendung nach einem der Ansprüche 4 bis 14, wobei die medizinische Vorrichtung ein biologisch abbaubares Polymer, wie z. B. Kollagen, Hyaluronsäure, Polymilchsäure oder Polyurethan, enthält oder daraus besteht.

## Revendications

1. Composition comprenant du fibrinogène à utiliser dans une méthode de traitement ou de prévention de la résistance antimicrobienne des micro-organismes chez l'homme ou l'animal.

2. Composition à utiliser selon la revendication 1, dans laquelle l'antimicrobien est un agent antibactérien, de préférence un antibiotique.

3. Composition à utiliser selon l'une des revendications précédentes, dans laquelle les micro-organismes se trouvent dans un biofilm ou sous une forme agrégée et la composition est destinée à augmenter la sensibilité antimicrobienne du biofilm ou de la forme agrégée.

4. Composition à utiliser selon l'une des revendications précédentes, dans laquelle le biofilm se trouve sur un dispositif médical ou sur le tissu d'une plaie.

5. Composition à utiliser selon l'une des revendications précédentes, dans laquelle la composition comprend du ou est constituée de fibrinogène plasmatique, fibrinogène de type sauvage, fibrinogène gamma prime, fibrinogène alpha variante étendue ou fibrinogène alpha variante tronquée.

6. Composition à utiliser selon l'une des revendications précédentes, dans laquelle le fibrinogène est utilisé pour recouvrir un dispositif médical ou un implant avant son implantation, ou une plaie avant pansement ou fermeture.

7. Composition à utiliser selon l'une des revendications précédentes, la composition comprenant en outre de la thrombine, de la batroxobine ou de la reptilase.

8. Composition à utiliser selon l'une des revendications précédentes, dans laquelle la méthode consiste en outre à fournir un agent antimicrobien.

9. Composition à utiliser selon la revendication 8, dans laquelle l'antimicrobien est inclus dans la composition.

10. Composition à utiliser selon l'une des revendications précédentes, dans laquelle le fibrinogène contenu dans la composition est produit par recombinaison.

11. Composition à utiliser selon l'une des revendications précédentes, dans laquelle les micro-organismes sont des bactéries Gram positives ou Gram négatives.

12. Composition à utiliser selon la revendication 11, dans laquelle les bactéries sont *Pseudomonas* espèces ou *Staphylococci, de préférence Staphylococcus aureus* espèces, plus préférentiellement 5. *aureus,* résistant à la vancomycine 5. *aureus,* résistant à la méthicilline 5. *aureus* ou multirésistants 5. *aureus* souches multirésistantes.

13. Composition à utiliser selon l'une des revendications précédentes, dans laquelle la composition est un spray, une pâte ou un gel.

14. Composition à utiliser selon l'une des revendications 4 à 13, dans laquelle le dispositif médical est un dispositif électronique implantable cardiaque (DEIC), un cathéter, une sonde endotrachéale, une sonde d'alimentation entérale, une valve cardiaque, un dispositif d'immobilisation, une prothèse articulaire, un stent, une trachéotomie, un dispositif d'accès vasculaire ou un drain de plaie.

15. Composition à utiliser selon l'une des revendications 4 à 14, dans laquelle le dispositif médical comprend ou est constitué d'un polymère biodégradable, tel que le collagène, l'acide hyaluronique, l'acide polylactique ou le polyuréthane.
